(19) Europäisches Patentamt

European Patent Office

Office européen des brevets

(11) **EP 3 423 799 B1**

(12) **EUROPEAN PATENT SPECIFICATION**

(45) Date of publication and mention
of the grant of the patent:
**08.07.2020 Bulletin 2020/28**

(21) Application number: **17711416.2**

(22) Date of filing: **28.02.2017**

(51) Int Cl.:
*G01K 7/42* *(2006.01)*    *G01K 3/00* *(2006.01)*
*G01K 1/02* *(2006.01)*    *G06Q 10/08* *(2012.01)*
*G01N 33/02* *(2006.01)*

(86) International application number:
**PCT/US2017/020005**

(87) International publication number:
**WO 2017/151656 (08.09.2017 Gazette 2017/36)**

(54) **SYSTEM AND METHOD OF REVERSE MODELING OF PRODUCT TEMPERATURES**

SYSTEM UND VERFAHREN ZUR REVERSEN MODELLIERUNG VON PRODUKTTEMPERATUREN

SYSTÈME ET PROCÉDÉ DE MODÉLISATION INVERSE DE TEMPÉRATURES DE PRODUIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.03.2016 US 201662301964 P**

(43) Date of publication of application:
**09.01.2019 Bulletin 2019/02**

(73) Proprietor: **Carrier Corporation
Palm Beach Gardens, FL 33418 (US)**

(72) Inventor: **CHERNEFF, Jonathan
Farmington, CT 06032 (US)**

(74) Representative: **Dehns
St. Bride's House
10 Salisbury Square
London EC4Y 8JD (GB)**

(56) References cited:
**WO-A1-2015/065899      WO-A2-03/012602
DE-A1-102005 003 562    US-A1- 2015 120 597**

## Description

### TECHNICAL FIELD OF THE DISCLOSED EMBODIMENTS

The presently disclosed embodiments generally relate to modeling temperatures, and more particularly, to a system and method reverse modeling of product temperatures

### BACKGROUND OF THE DISCLOSED EMBODIMENTS

Many products, such as refrigerated products, have temperature requirements. It can be prohibitively expensive in terms of both time and money to place thermometers within, or in contact with, the products themselves. Therefore, temperature measurements are typically taken of the surrounding ambient air. However, measuring the temperature of the ambient air can be a poor substitute for the information that is actually desired, i.e., the temperature of the product.

Moreover, the process of accepting or rejecting food shipments typically involves taking a temperature of the product via a temperature probe; however, that alone does not indicate the probable temperature history of the product. There is therefore a need for an improved method of modeling product temperature to determine whether shipments should be accepted or rejected. An example of a method of reverse modelling temperature history of a product during shipping can be found in DE 10 2005 003 562 A1.

### SUMMARY OF THE DISCLOSED EMBODIMENTS

In one aspect, a method of estimating a temperature history of a product transported in a shipping enclosure is provided. The method includes measuring an actual ending temperature of the product, acquiring ambient temperature data of the shipping enclosure during the duration of a trip, creating a plurality of simulated trips comprising simulated product temperature histories based in part on the ambient air temperature data of the shipping enclosure, wherein each of the plurality of simulated product temperature histories includes an estimated ending product temperature value, determining which of the simulated product temperature histories produce an estimated ending product temperature value wherein the difference between the estimated ending product temperature value and the actual ending temperature of the product is within a temperature threshold; thereby determining which of the plurality of simulated trips to select to provide an estimation of the temperature history of the product for the duration of the trip. In one embodiment, the temperature threshold is adjustable.

In one embodiment, measuring an actual temperature of the product includes measuring the actual temperature of the product upon arrival at a destination other than the origin. In one embodiment, creating a plurality of simulated trips based in part on the ambient air temperature data of the shipping enclosure includes determining at least one of a thermal mass and a thermal conductivity of the product.

In one aspect, a system for estimating a temperature history of a product transported in a shipping container is provided. The system includes a network, a first data recordation device in communication with the network, wherein the first data recordation device is configured to acquire ambient temperature data of the shipping enclosure, and a remote computing device in communication with the network, wherein the remote computing device is configured to create a plurality of simulated trips based in part on the ambient air temperature data of the shipping enclosure, wherein each of the plurality of simulated trips includes an estimated ending product temperature value.

In this embodiment, the system further includes a second data recordation device in communication with the network, wherein the second data recordation device is configured to measure an actual temperature of the product. The remote computing device is further configured to determine whether the difference between each of the estimated ending product temperature values and the actual temperature of the product is within a temperature threshold to thereby determine which of the plurality of simulated trips to select in order to provide an estimation of the temperature history of the product for the duration of the trip. In an embodiment, the temperature threshold is adjustable.

### BRIEF DESCRIPTION OF DRAWINGS

FIG. 1 illustrates a schematic diagram of a modeling system according to an embodiment of the present disclosure;
FIG. 2 illustrates a schematic flow diagram of a method to reverse model product temperatures according to an embodiment of the present disclosure;
FIG. 3 illustrates a graph depicting a reverse model of product temperatures according to an embodiment of the present disclosure; and
FIG. 4 illustrates a graph depicting a reverse model of product temperatures according to an embodiment of the present disclosure.

### DETAILED DESCRIPTION OF THE DISCLOSED EMBODIMENTS

For the purposes of promoting an understanding of the principles of the present disclosure, reference will now be made to the embodiments illustrated in the drawings, and specific language will be used to describe the same. It will nevertheless be understood that no limitation of the scope of this disclosure is thereby intended.

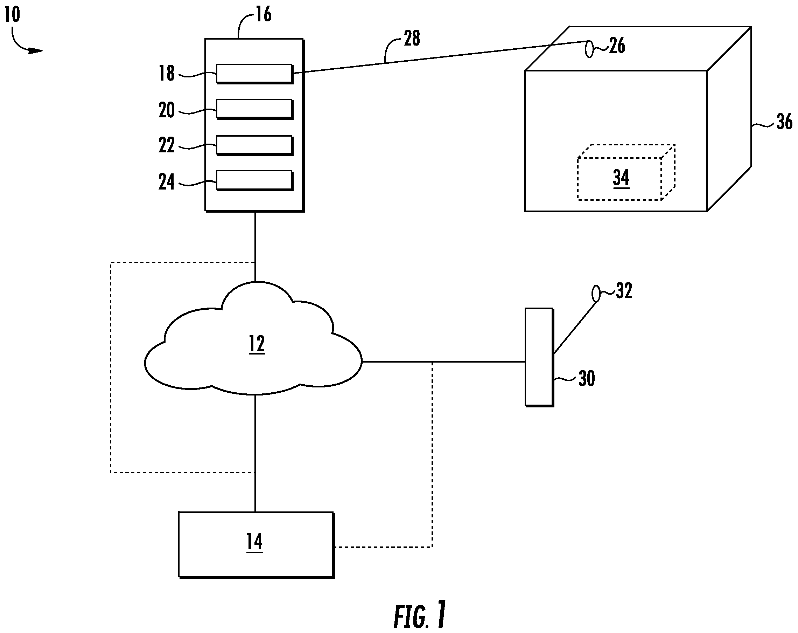

FIG. 1 schematically illustrates an embodiment of a product temperature measuring system, generally indicated at 10. In the embodiment shown, the system 10 includes a network 12 in communication with a remote computing device 14 and a first data recordation device 16. It will be appreciated that the remote computing device 14 may be in direct communication with the first data recordation device 16 via a wired or wireless connection.

The data recordation device 16 includes an intake engine 18, a calculation engine 20, an interface engine 22, a database 24, and a first temperature measuring device 26 communicatively connected with intake engine 18 via a wire 28. It will be appreciated that the first temperature measuring device 26 may be communicatively connected to the data recordation device 16 via any means known in the art, such as wirelessly. The data recordation device 16 is configured to measure an ambient air temperature within a shipping enclosure (not shown), for example, a refrigerated truck to name one non-limiting example. In the embodiment shown, the first temperature measuring device 26 is placed on a product container 36 within the shipping enclosure. It will be appreciated that the first temperature measuring device 26 may be placed anywhere within the shipping enclosure suitable to measure the ambient air temperature therein.

The system 10 further includes a second data recordation device 30 in communication with the network 12. The second data recordation device 30 includes a second temperature measuring device 32, and is configured to measure an actual temperature of a product 34 stored and transported in a product container 36. The second data recordation device 30 is typically located at one or more of the locations along the route of the shipping enclosure. It will be appreciated that the remote computing device 14 may be in direct communication with the second data recordation device 30 via a wired or wireless connection. The system 10 is configured to estimate a temperature history of the product 34 stored and transported in the product container 36 based in part on the method described herein.

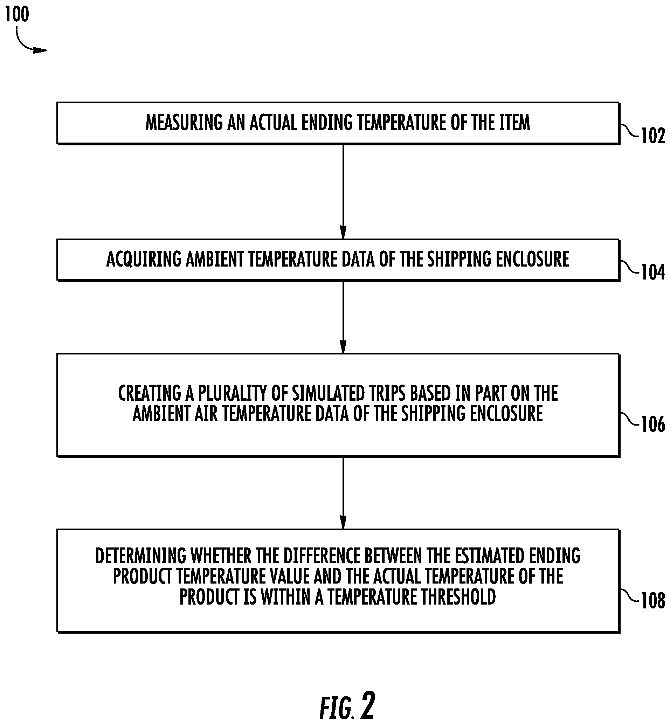

FIG. 2 illustrates a method of estimating a temperature history of the product 34 stored and transported in the shipping enclosure, the method generally indicated at 100. The method 100 includes step 102 of measuring an actual temperature of the product 34. In an embodiment, measuring an actual temperature of the product 34 includes measuring the actual temperature of the product 34 upon arrival at a destination other than the origin. With reference to FIG. 1, upon arrival at a destination, for example a final destination, of the shipment of product 34, a user may measure the temperature of the product 34 utilizing the second temperature measuring device 32 of the second data recordation device 30.

The method 100 further includes step 104 of acquiring ambient temperature data of the shipping enclosure. In an embodiment, step 104 includes operating the first data recordation device 16 to measure ambient air temperature data within the shipping enclosure for a trip duration time.

The method 100 further includes step 106 of creating a plurality of simulated trips based in part on the ambient air temperature data of the shipping enclosure, wherein each of the plurality of simulated trips includes an estimated ending product temperature value. In an embodiment, step 106 further includes determining at least one of a thermal mass, a thermal conductivity, and thermal characteristics of the product 34. For example, a user may operate the remote computing device 14 to select the type of product 34 being transported, for example, a specific type of meat, produce, ice cream, etc. to name a few non-limiting examples. The remote computing device 14 may either store in memory or download from the network 12 the thermal mass and/or thermal conductivity and/or other thermal characteristics of the product 34.

The method 100 further includes step 108 of determining whether the difference between the estimated ending product temperature value and the actual temperature of the product 34 is within a temperature threshold. In one embodiment, the temperature threshold is adjustable.

Working through an example, for illustrative purposes only, before the product 34 leaves its origin, a user may operate the first data recordation device 16 to begin collecting ambient air temperature data within the shipping enclosure. During the duration of the trip, the first data recordation device 16 measures and records the ambient air temperature within the shipping enclosure. Once the product 34 arrives at the desired destination, another user may operate the first data recordation device 16 to transmit the ambient air temperature data for the duration of the trip to the remote computing device 14 over the network 12.

In an embodiment, once the remote computing device 14 receives the ambient air temperature data from the first data recordation device 16 and the actual temperature of the product 34 upon arrival, the remote computing device 14 selects an estimated starting product temperature value to begin a simulated trip. Based on the estimated starting product temperature value, the remote computing device 14 utilizes the following calculation to produce an estimated temperature difference between the product and the ambient air.

$$\Delta T_{n+1} = k(A_n - T_n)$$

Where $A_n$ is the ambient air temperature value at a given time, $T_n$ is the estimated temperature of the product at the given time, $k$ is the thermal conductivity and/or the thermal mass of the product 34.

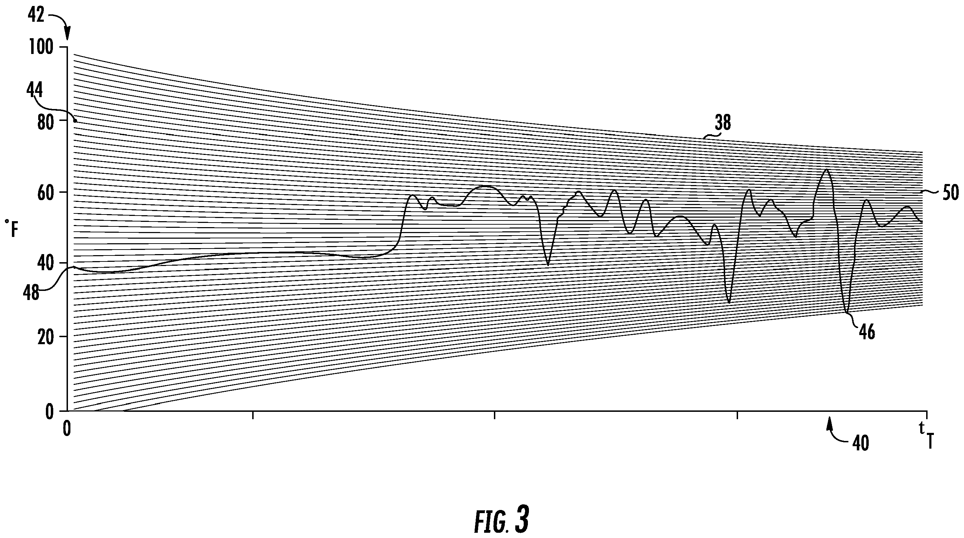

An example of a plurality of simulated trips 38 is shown in FIG. 3. The x-axis 40 of the graph is time, and the y-axis 42 is the estimated temperature of the product 34 in degrees Fahrenheit (° F). To estimate the temperature history of the product 34, for illustrative purposes only, the remote computing device 14 first selects an estimated starting product temperature value, for example approximately 80° F (designated at point 44). Assuming a thermal conductivity and thermal mass value $k$ of approximately 0.0375, the remote computing device 14 takes the difference between the ambient air temperature value, as shown by the line 46, and the estimated starting product temperature value at the next time interval (i.e., $t_1$).

In the example shown, the ambient air temperature value at time $t_1$, designated at point 48 is approximately 39° F. As such, the remote computing device 14 then estimates the temperature difference of the product 34 at time $t_2$ to be approximately -1.5375° F, resulting in an estimated product temperature at time $t_2$ of approximately 78.4625° F. The remote computing device 14 will continue to estimate the temperature of the product 34 for the time period equivalent to the entire duration of the trip until an estimated ending product temperature is determined (e.g. approximately 63° F for a starting temperature of approximately 80° F).

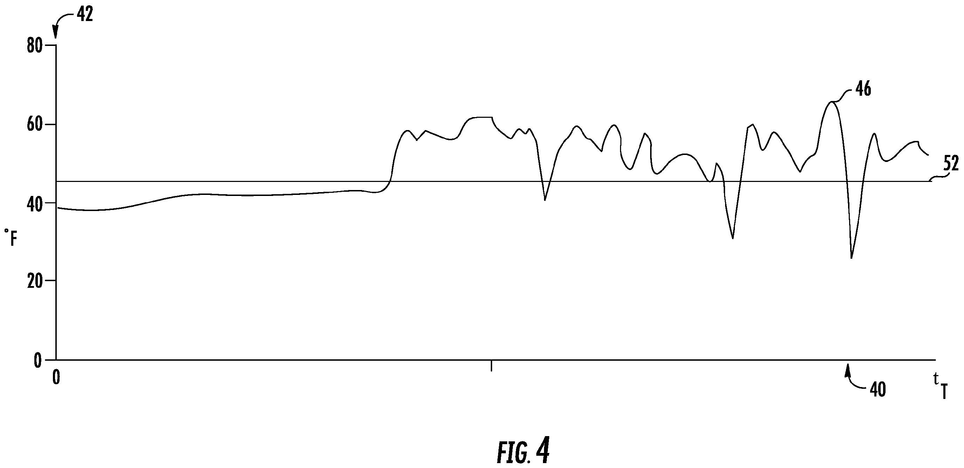

If the actual temperature of the product 34 is measured at approximately 44° F at the end of the trip, and the temperature threshold is established as $\pm$ 1°F, the remote computing device 14 determines which of the simulated trips produces an estimated ending product temperature of approximately 44° F $\pm$ 1° F. As shown in FIG.4, the remote computing device determines that the simulated trip 52 produces an estimated ending product temperature of approximately 44° F $\pm$ 1° F. As such, a user may examine the simulated trip 38' to estimate the temperature history of the product 34 for the duration of trip.

It will therefore be appreciated that the present system 10 and method 100 allows a user to estimate the temperature history of the product 34 by using the ambient air temperature data, the actual temperature of the product 34 upon arrival at the final destination; thus providing the user with valuable information and allowing the user to make better judgments on the acceptance or denial of the product based on the temperature history of the product 34.

## Claims

1. A method of estimating a temperature history of a product transported in a shipping enclosure, the method comprising:

   (a) measuring an actual ending temperature of the product;
   (b) acquiring ambient air temperature data of the shipping enclosure during the duration of a trip;
   (c) creating a plurality of simulated trips comprising simulated product temperature histories based in part on the ambient air temperature data of the shipping enclosure, wherein each of the plurality of simulated product temperature histories includes an estimated ending product temperature value;
   (d) determining which of the simulated product temperature histories produce an estimated ending product temperature value wherein the difference between the estimated ending product temperature value and the actual ending temperature of the product is within a temperature threshold; and
   (e) thereby determining which of the plurality of simulated trips to select to provide an estimation of the temperature history of the product for the duration of the trip.

2. The method of claim 1, wherein step (a) comprises measuring the actual temperature of the product upon arrival at a destination other than the origin.

3. The method of claim 1, wherein step (c) further comprises determining at least one of a thermal mass and a thermal conductivity of the product.

4. The method of claim 1, wherein the temperature threshold is adjustable.

5. A system for estimating a temperature history of a product transported in a shipping container comprising:

   a network (12);
   a first data recordation device (16) in communication with the network (12), wherein the first data recordation device (16) is configured to acquire ambient air temperature data of the shipping enclosure; a remote computing device (14) in communication with the network (12), wherein the remote computing device (14) is configured to create a plurality of simulated trips based in part on the ambient air temperature data of the shipping enclosure, wherein each of the plurality of simulated trips includes an estimated ending product temperature value; and
   a second data recordation device (30) in communication with the network (12), wherein the second data recordation device (130) is configured to measure an actual ending temperature of the product;
   wherein the remote computing device (14) is further configured to determine whether the difference between each of the estimated ending product temperature values and the actual ending temperature of the product is within a temperature threshold to thereby determine which of the plurality of simulated trips to select in order to provide an estimation of the temperature history of the product for the duration of the trip.

**6.** The system of claim 5, wherein the temperature threshold is adjustable.

**Patentansprüche**

**1.** Verfahren zum Schätzen eines Temperaturverlaufs eines Produkts, das in einer Versandhülle transportiert wird, wobei das Verfahren Folgendes umfasst:

(a) Messen einer tatsächlichen Endtemperatur des Produkts;
(b) Erfassen von Umgebungslufttemperaturdaten der Versandhülle während der Dauer einer Fahrt;
(c) Erzeugen einer Vielzahl von simulierten Fahrten, die simulierte Produkttemperaturverläufe teilweise auf Grundlage der Umgebungslufttemperaturdaten der Versandhülle umfassen, wobei jeder von der Vielzahl von simulierten Produkttemperaturverläufen einen geschätzten Endprodukttemperaturwert beinhaltet;
(d) Bestimmen, welcher der simulierten Produkttemperaturverläufe einen geschätzten Endprodukttemperaturwert erzeugt, wobei die Differenz zwischen dem geschätzten Endprodukttemperaturwert und dem tatsächlichen Endprodukttemperatur des Produkts innerhalb eines Temperaturschwellenwerts liegt; und
(e) dadurch Bestimmen, welche von der Vielzahl von simulierten Fahrten auszuwählen ist, um eine Schätzung des Temperaturverlaufs des Produkts für die Dauer der Fahrt bereitzustellen.

**2.** Verfahren nach Anspruch 1, wobei Schritt (a) das Messen der tatsächlichen Temperatur des Produkts bei der Ankunft an einem Zielort, der sich von dem Ausgangsort unterscheidet, umfasst.

**3.** Verfahren nach Anspruch 1, wobei Schritt (c) ferner das Bestimmen von mindestens einem von einer thermischen Masse und einer thermischen Leitfähigkeit des Produkts umfasst.

**4.** Verfahren nach Anspruch 1, wobei der Temperaturschwellenwert einstellbar ist.

**5.** System zum Schätzen eines Temperaturverlaufs eines Produkts, das in einer Versandhülle transportiert wird, umfassend:

ein Netzwerk (12);
eine erste Datenaufzeichnungsvorrichtung (16) in Kommunikation mit dem Netzwerk (12), wobei die erste Datenaufzeichnungsvorrichtung (16) dazu ausgelegt ist, Umgebungslufttemperaturdaten der Versandhülle zu erfassen; eine entfernte Rechenvorrichtung (14) in Kommunikation mit dem Netzwerk (12), wobei die entfernte Rechenvorrichtung (14) dazu ausgelegt ist, eine Vielzahl von simulierten Fahrten teilweise auf Grundlage der Umgebungslufttemperaturdaten der Versandhülle zu erzeugen, wobei jede von der Vielzahl von simulierten Fahrten einen geschätzten Endprodukttemperaturwert beinhaltet; und
eine zweite Datenaufzeichnungsvorrichtung (30) in Kommunikation mit dem Netzwerk (12), wobei die zweite Datenaufzeichnungsvorrichtung (130) dazu ausgelegt ist, eine tatsächliche Endtemperatur des Produkts zu messen;
wobei die entfernte Rechenvorrichtung (14) ferner dazu ausgelegt ist, zu bestimmen, ob die Differenz zwischen jedem von den geschätzten Endprodukttemperaturwerten und der tatsächlichen Endtemperatur des Produkts innerhalb eines Temperaturschwellenwerts liegt, um dadurch zu bestimmen, welche von der Vielzahl von simulierten Fahrten auszuwählen ist, um eine Schätzung des Temperaturverlaufs für die Dauer der Fahrt bereitzustellen.

**6.** System nach Anspruch 5, wobei der Temperaturschwellenwert einstellbar ist.

**Revendications**

**1.** Procédé d'estimation d'un historique de température d'un produit transporté dans une enceinte d'expédition, le procédé comprenant :

(a) la mesure d'une température finale réelle du produit ;
(b) l'acquisition de données sur la température de l'air ambiant de l'enceinte d'expédition pendant la durée d'un trajet ;
(c) la création d'une pluralité de trajets simulés comprenant des historiques de température de produits simulés en partie sur la base des données sur la température de l'air ambiant de l'enceinte d'expédition, dans lequel chacun de la pluralité d'historiques de température de produit simulé comporte une valeur de température de produit finale estimée ;
(d) la détermination desquels des historiques de température de produit simulés produisent une valeur de température de produit finale estimée dans lequel la différence entre la valeur de température de produit finale estimée et la température finale réelle du produit est dans un seuil de température ; et
(e) la détermination en résultant duquel des trajets simulés sélectionner parmi la pluralité de trajets simulés pour fournir une estimation de l'historique de température du produit pendant la durée du trajet.

2. Procédé selon la revendication 1, dans lequel l'étape (a) comprend la mesure de la température réelle du produit à son arrivée à une destination autre que l'origine.

3. Procédé selon la revendication 1, dans lequel l'étape (c) comprend en outre la détermination d'au moins l'une d'une masse thermique et d'une conductivité thermique du produit.

4. Procédé selon la revendication 1, dans lequel le seuil de température est réglable.

5. Système d'estimation d'un historique de température d'un produit transporté dans un conteneur d'expédition comprenant : un réseau (12) ;
un premier dispositif d'enregistrement de données (16) en communication avec le réseau (12), dans lequel le premier dispositif d'enregistrement de données (16) est configuré pour acquérir des données de température de l'air ambiant de l'enceinte d'expédition ;
un dispositif informatique distant (14) en communication avec le réseau (12), dans lequel le dispositif informatique distant (14) est configuré pour créer une pluralité de trajets simulés sur la base en partie des données de température de l'air ambiant de l'enceinte d'expédition, dans lequel chacun de la pluralité de trajets simulés comporte une valeur de température de produit finale estimée ; et
un second dispositif d'enregistrement de données (30) en communication avec le réseau (12), dans lequel le second dispositif d'enregistrement de données (130) est configuré pour mesurer une température finale réelle du produit ;
dans lequel le dispositif informatique distant (14) est en outre configuré pour déterminer si la différence entre chacune des valeurs de température de produit finale estimée et la température finale réelle du produit est dans un seuil de température pour ainsi déterminer lequel des trajets simulés sélectionner parmi la pluralité de trajets simulés pour fournir une estimation de l'historique de température du produit pendant la durée du trajet.

6. Système selon la revendication 5, dans lequel le seuil de température est réglable.

FIG. 1

EP 3 423 799 B1

100

MEASURING AN ACTUAL ENDING TEMPERATURE OF THE ITEM ~102

ACQUIRING AMBIENT TEMPERATURE DATA OF THE SHIPPING ENCLOSURE ~104

CREATING A PLURALITY OF SIMULATED TRIPS BASED IN PART ON THE AMBIENT AIR TEMPERATURE DATA OF THE SHIPPING ENCLOSURE ~106

DETERMINING WHETHER THE DIFFERENCE BETWEEN THE ESTIMATED ENDING PRODUCT TEMPERATURE VALUE AND THE ACTUAL TEMPERATURE OF THE PRODUCT IS WITHIN A TEMPERATURE THRESHOLD ~108

FIG. 2

FIG. 3

FIG. 4

EP 3 423 799 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102005003562 A1